(19) Europäisches Patentamt
European Patent Office
Office européen des brevets

(11) Veröffentlichungsnummer: **0 117 229**
**A1**

(12) # EUROPÄISCHE PATENTANMELDUNG

(21) Anmeldenummer: **84810055.8**

(22) Anmeldetag: **30.01.84**

(51) Int. Cl.³: **C 07 D 401/14**
C 08 K 5/34

(30) Priorität: **04.02.83 IT 1943083**

(43) Veröffentlichungstag der Anmeldung:
**29.08.84 Patentblatt 84/35**

(84) Benannte Vertragsstaaten:
**DE FR GB IT**

(71) Anmelder: **CIBA-GEIGY S.p.A.**
**Strada Statale 233-Km. 20,5**
**Origgio(IT)**

(72) Erfinder: **Cantatore, Giuseppe, Dr.**
**Via Generale Pianelli, 68**
**I-70032 Bitonto (Bari)(IT)**

(74) Vertreter: **Stamm, Otto et al,**
**Patentabteilung der CIBA-GEIGY AG Postfach**
**CH-4002 Basel(CH)**

(54) **Neue, als Stabilisatoren für synthetische Polymere verwendbare Piperidinyl-triazinverbindungen.**

(57) Neue, als Licht-, Wärme- und Oxydationsstabilisatoren für synthetische Polymere verwendbare Piperidinyl-triazinverbindungen entsprechen der allgemeinen Formel:

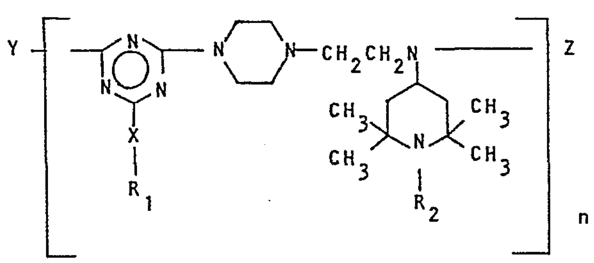

worin $R_1$ und $R_2$ für H, Alkyl oder sonstige im Text definierte Reste sowie Y und Z für im Text definierte Endgruppen stehen.

**EP 0 117 229 A1**

# Neue, als Stabilisatoren für synthetische Polymere verwendbare Piperidinyl-triazinverbindungen

Vorliegende Erfindung betrifft neue Piperidinyl-triazinverbindungen, das Verfahren zu deren Herstellung und ihre Verwendung als Licht-, Wärme- und Oxydationsstabilisatoren für synthetische Polymere.

Gegenstand der neuen Erfindung sind insbesondere neue Verbindungen der allgemeinen Formel (I)

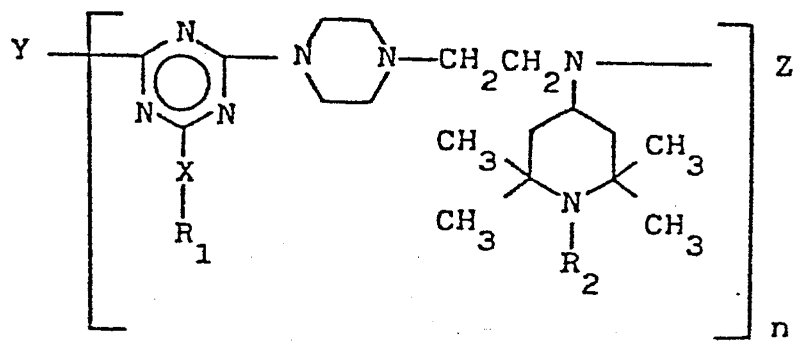

(I)

worin $R_1$ für Wasserstoff, $C_1$-$C_{18}$-Alkyl, $C_3$-$C_{18}$-Alkenyl, $C_5$-$C_{18}$-Cycloalkyl, unsubstituiertes oder durch 1 bis 3 $C_1$-$C_8$-Alkylreste substituiertes Phenyl, unsubstituiertes oder durch 1 bis 3 $C_1$-$C_8$-Alkylreste substituiertes Hydroxyphenyl, unsubstituiertes oder durch 1 bis 3 $C_1$-$C_4$-Alkylreste substituiertes Benzyl oder einen Rest $-R_3-Z'$, in dem $R_3$ $C_2$-$C_6$-Alkylen und Z' $-OR_4$ oder $-\underset{R_5}{\underset{|}{N}}-R_6$ bedeuten, wobei $R_4$ Wasserstoff oder $C_1$-$C_{18}$-Alkyl sowie $R_5$ und $R_6$, die gleich oder verschieden sein können, $C_1$-$C_8$-Alkyl oder $C_3$-$C_8$-Alkenyl darstellen oder $-\underset{R_5}{\underset{|}{N}}-R_6$ als

ganzes Morpholin-4-yl, 4-Methyl-piperazin-1-yl oder 2,2,6,6-Tetramethyl-piperidin-1-yl sein kann, oder für einen Rest der Formel (II)

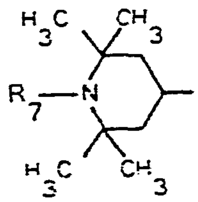

(II)

steht, worin $R_7$ Wasserstoff, O·, Cyanmethyl, $C_1$-$C_{12}$-Alkyl, $C_3$-$C_{12}$-Alkenyl oder -alkinyl, unsubstituiertes oder durch 1 bis 3 $C_1$-$C_4$-Alkylreste substituiertes Benzyl, unsubstituiertes oder durch 1 bis 3 $C_1$-$C_4$-Alkylreste substituiertes Hydroxybenzyl, $C_1$-$C_{12}$-Acyl, 2,3-Epoxypropyl oder $-CH_2-\underset{R_8}{\underset{|}{C}}H-OR_9$ bedeutet, worin $R_8$ Wasserstoff, Methyl, Aethyl oder Phenyl und $R_9$ Wasserstoff, $C_1$-$C_{12}$-Alkyl, $C_3$-$C_{12}$-Alkenyl, $C_7$-$C_{12}$-Aralkyl oder $C_1$-$C_{12}$-Acyl darstellen, $R_2$ die für $R_7$ angegebene Bedeutung hat, X für -O- oder $-\underset{R_{10}}{\underset{|}{N}}-$ steht, wobei $R_{10}$ die für $R_1$ angegebene Bedeutung hat, oder $R_1X$- als ganzes auch für Chlor, Pyrrolidin-1-yl, Piperidin-1-yl, Hexahydroazepin-1-yl, Morpholin-4-yl, 4-Methyl-piperazin-1-yl oder einen Rest der Formel (IIIa) oder (IIIb)

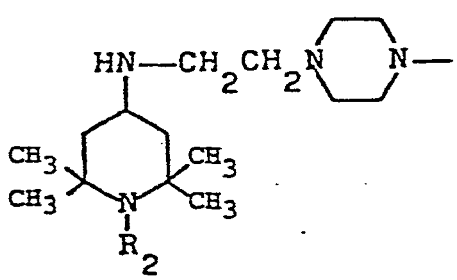

(IIIa)

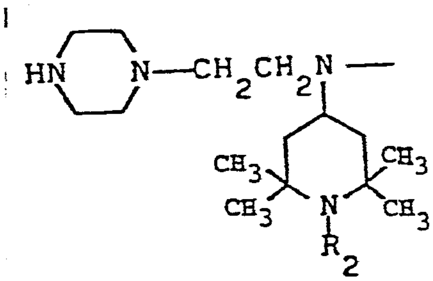

(IIIb)

stehen kann, worin $R_2$ die oben angegebene Bedeutung hat, Y für Chlor, einen Rest $R_1X$-, worin $R_1$ und X die oben angegebenen Bedeutungen haben, oder einen Rest der Formel (IIIa) oder (IIIb) und Z für Wasserstoff oder einen Rest der Formel (IV)

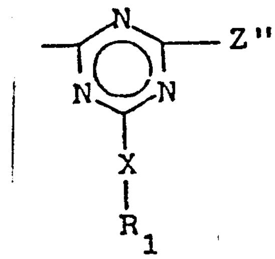

(IV)

stehen, worin Z" Chlor oder einen Rest $R_1X$- darstellt, worin $R_1$ und X die oben angebebenen Bedeutungen haben, und n eine ganze Zahl von 1 bis 100 ist.

Erläuternde Beispiele für die Bedeutungen der verschiedenen Reste in der Formel (I) sind wie folgt:

für $R_1$ und $R_{10}$: Wasserstoff, Methyl, Aethyl, Propyl, Isopropyl, Butyl, 2-Butyl, Isobutyl, Hexyl, 2-Aethylhexyl, Octyl, Decyl, Dodecyl, Hexadecyl, Octadecyl, Allyl, Methallyl, But-2-enyl, Undec-10-enyl, Oleyl, Cyclohexyl, 2- oder 4-Methylcyclohexyl, 3,3,5-Trimethylcyclohexyl, Cyclooctyl, Cyclododecyl, Phenyl, o-, m- und p-Methylphenyl, 2,6-Dimethylphenyl, 2,4,6-Trimethylphenyl, 4-t-Butylphenyl, 4-t-Octylphenyl, 4-Methoxyphenyl, 4-Aethoxyphenyl, 3,5-Di-t-butyl-4-hydroxyphenyl, Benzyl, 4-Methylbenzyl, 4-Hydroxybenzyl, 3,5-Di-t-butyl-4-hydroxybenzyl, 2,2,6,6-Tetramethylpiperidin-4-yl, 1,2,2,6,6-Pentamethyl-piperidin-4-yl, 1-Allyl-2,2,6,6-tetramethyl-piperidin-4-yl, 1-Benzyl-2,2,6,6-tetramethyl-piperidin-4-yl und 1-Acetyl-2,2,6,6-tetramethyl-piperidin-4-yl;

für $R_2$ und $R_7$: Wasserstoff, Cyanmethyl, Methyl, Aethyl, Propyl, Butyl, Hexyl, Octyl, Decyl, Dodecyl, Allyl, Methallyl, But-2-enyl, Hex-2-enyl, Undec-10-enyl, Propargyl, Benzyl, 4-Methylbenzyl, 4-t-Butylbenzyl, 4-Hydroxybenzyl, Acetyl, Propionyl, Butyryl, Caproyl, Benzoyl, 2,3-Epoxypropyl und 2-Hydroxyäthyl;

für $R_3$: Aethylen, 1,2- oder 1,3-Propylen, Tetramethylen und Hexamethylen;

für $R_4$: Wasserstoff, Methyl, Aethyl, Propyl, Butyl, Hexyl, Heptyl, Octyl, 2-Aethylhexyl, Decyl, Dodecyl, Hexadecyl und Octadecyl;

für $R_5$ und $R_6$: Methyl, Aethyl, Propyl, Butyl, Hexyl, Octyl, Allyl, But-2-enyl und Hex-2-enyl;

für $R_9$: Wasserstoff, Methyl, Aethyl, Propyl, Butyl, Hexyl, Heptyl, Octyl, 2-Aethylhexyl, Decyl, Dodecyl, Allyl, Methallyl, But-2-enyl, Hex-2-enyl, Undec-10-enyl, Benzyl, 4-Methylbenzyl, 4-t-Butylbenzyl, Acetyl, Propionyl, Butyryl, Caproyl und Benzoyl.

Bevorzugt werden solche Verbindungen der Formel (I), in denen $R_1$ und $R_{10}$, die gleich oder verschieden sein können, für Wasserstoff, $C_1$-$C_{12}$-Alkyl, $C_6$-$C_{12}$-Cycloalkyl, 2,2,6,6-Tetramethyl-piperidin-4-yl oder 1,2,2,6,6-Pentamethyl-piperidin-4-yl, $R_2$ und $R_7$, die gleich oder verschieden sein können, für Wasserstoff, $C_1$-$C_6$-Alkyl, $C_3$-$C_6$-Alkenyl oder -alkinyl, Benzyl, $C_1$-$C_6$-Alkanoyl, 2,3-Epoxypropyl oder 2-Hydroxyäthyl oder $R_1X$- als ganzes für Chlor, Piperidin-1-yl, Hexahydroazepin-1-yl, Morpholin-4-yl oder einen Rest der Formel (IIIa) oder (IIIb) stehen und n eine ganze Zahl von 1 bis 50 ist.

Solche Verbindungen der Formel (I) werden besonders bevorzugt, in denen $R_1$ für Wasserstoff, $C_1$-$C_8$-Alkyl, 2,2,6,6-Tetramethyl-piperidin-4-yl oder 1,2,2,6,6-Pentamethyl-piperidin-4-yl, $R_2$ und $R_7$ für Wasserstoff oder Methyl und $R_{10}$ für Wasserstoff oder $C_1$-$C_8$-Alkyl stehen, n eine ganze Zahl von 1 bis 20 ist und Y für einen Rest $R_1X$- oder einen Rest (IIIa) oder (IIIb) und Z für Wasserstoff oder einen Rest

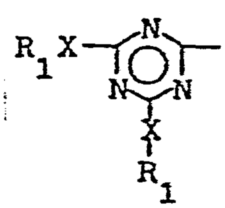

stehen.

Eine Verbindung der Formel (I) lässt sich dadurch herstellen, dass man eine Verbindung der Formel (V)

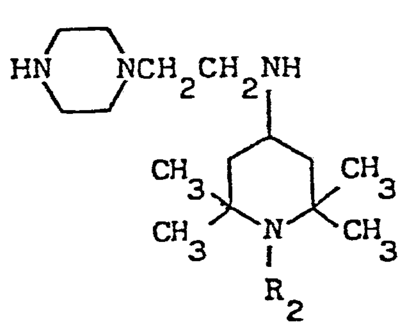

(V)

mit Cyanurchlorid oder dessen teilweise substituierten Derivaten der Formel (VI) oder (VII)

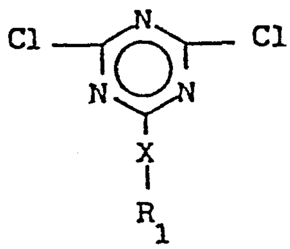

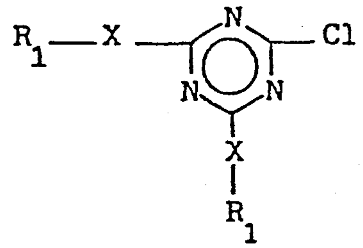

(VI) (VII)

worin $R_1X$- von Chlor verschieden ist, umsetzt.

Gemäss einer ersten Variante setzt man eine Verbindung der Formel (V) mit Cyanurchlorid in einem inerten Lösungsmittel und in Gegenwart einer organischen oder anorganischen Base bei einer Temperatur zwischen -20° und 100°C, vorzugsweise zwischen 0° und 80°C, in einem Molverhältnis der Verbindung der Formel (V) zu Cyanurchlorid zwischen 1:2 und 2:1, vorzugsweise zwischen 1:2 und 1:1, um, wobei man Verbindungen der Formel (VIII)

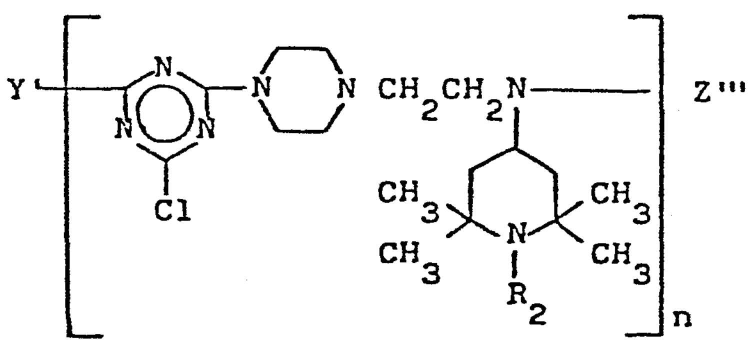

(VIII)

erhält, worin Y' für Chlor oder einen Rest der Formel (IIIa) oder (IIIb) und Z"' für Wasserstoff oder einen Rest der Formel (IX)

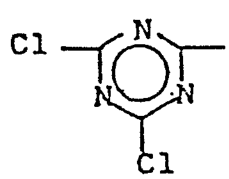

(IX)

stehen und n die oben angegebene Bedeutung hat.

Anschliessend werden die Verbindungen der Formel (VIII) mit Verbindungen der Formel (X)

$$R_1-X-H \qquad (X)$$

in dem Cyanurchlorid mindestens äquimolarer Menge umgesetzt; diese Reaktion erfolgt im selben Reaktor bei einer Temperatur zwischen 50° und 200°C, vorzugsweise zwischen 80° und 180°C, wobei man Verbindungen der Formel (I) erhält.

Gemäss einer zweiten Variante setzt man eine Verbindung der Formel (V) mit einer Verbindung der Formel (VI) in einem inerten Lösungsmittel und in Gegenwart einer organischen oder anorganischen Base bei einer Temperatur zwischen 50° und 200°C, vorzugsweise zwischen 80° und 180°C, in einem Molverhältnis der Verbindung der Formel (V) zur Verbindung der Formel (VI) zwischen 1:2 und 2:1, vorzugsweise zwischen 1,2:1 und 1:1,2, zu Verbindungen der Formel (I) um.

Gemäss einer dritten Variante setzt man eine Verbindung der Formel (V) mit einer Verbindung der Formel (VII) in einem inerten Lösungsmittel und in Gegenwart einer organischen oder anorganischen Base bei Temperaturen zwischen 50° und 200°C, vorzugsweise zwischen 80° und 180°C, in einem Molverhältnis der Verbindung der Formel (V) zur Verbindung der Formel (VII) von 1:2 zu Verbindungen der Formel (I) um.

Bei den möglichen Varianten kann die Herstellung der Verbindungen der Formel (I) jeweils so erfolgen, dass man von Cyanurchlorid ausgeht, das direkt oder nach Ueberführung in seine im selben Reaktor herstellbare und ohne Isolierung aus dem Reaktionsgemisch verwendbare Derivate der Formeln (VI) und (VII) eingesetzt wird; es ist jedoch ebenfalls möglich, getrennt hergestellte Verbindungen der Formel (VI) und (VII) zusammen mit den Verbindungen der Formel (V) in derselben Reaktion zu verwenden.

Als Reaktionsmedium kann man ein einziges inertes Lösungsmittel, zum Beispiel Aceton, Methyläthylketon, Methylisobutylketon, Aether, Tetrahydrofuran, Dioxan, Benzol, Toluol, Xylol, Trimethylbenzol, Aethylbenzol, Tetralin, Decalin, Octan oder Decan, verwenden.

Um die bei dem Verfahren freigesetzte Salzsäure zu binden, wird die Umsetzung jeweils in Gegenwart von organischen oder anorganischen Basen, beispielsweise Triäthylamin,

Tributylamin, Natrium- oder Kaliumhydroxyd oder Natrium- oder Kaliumcarbonat durchgeführt, oder im Fall, wo -X- für -O- steht, wird das Reagens der Formel (X) in Form des Alkoholats eines Alkalimetalls verwendet.

In jedem Fall wird die Base in einer der während der Reaktion freigesetzten Salzsäure mindestens äquivalenten Menge eingesetzt.

Die Verbindungen der Formel (V), d.h. die Ausgangsstoffe für die Herstellung der Verbindungen der Formel (I), sind neu und lassen sich durch reduktive Alkylierung von 1-(2-Aminoäthyl)-piperazin mit Piperidonen der Formel (XI)

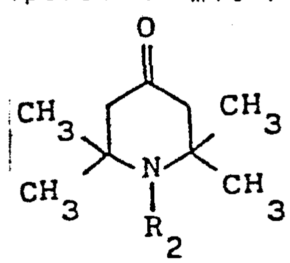

(XI)

in Gegenwart von Hydrierungskatalysatoren herstellen.

Zur näheren Erläuterung der vorliegenden Erfindung werden unten einige Herstellungsbeispiele für die Verbindungen der Formel (I) beschrieben, wobei diese Beispiele zur Erläuterung dienen und in keiner Weise als Beschränkungen auszulegen sind.

BEISPIEL 1 - Herstellung von 1-[2-(2,2,6,6-Tetramethyl-piperidin-4-ylamino)-äthyl]-piperazin.

Man beschickt einen 2-Liter Autoklaven mit 465 g (3 Mol) Triacetonamin, 200 ml Isopropanol, 387 g (3 Mol) N-(2-Aminoäthyl)-piperazin und 3 g Platinkatalysator (5% auf Kohle).

Nach Spülung mit Stickstoff wird Wasserstoff auf 40 bar eingepresst und der Autoklav auf 80° - 90°C erhitzt, bis die Reaktion vollständig ist.

Nach dem Abkühlen wird der Wasserstoff abgeblasen, der Katalysator durch Filtrieren abgetrennt, das Lösungsmittel verdampft und das Produkt durch Destillation gereinigt: Siedepunkt 136-8°C/0,5 mm Hg.

Analyse für $C_{15}H_{32}N_4$

berechnet: C 67,11% H 12,02% N 20,87%
gefunden: C 66,39% H 12,05% N 20,70%

BEISPIEL 2

Man erhitzt 42,4 g (0,1 Mol) 2-Chlor-4,6-bis-(2,2,6,6-tetramethyl-piperidin-4-ylamino)-1,3,5-triazin, 13,4 g (0,05 Mol) 1-[2-(2,2,6,6-Tetramethyl-piperidin-4-yl-amino)-äthyl]-piperazin, 100 ml Xylol und 6 g Natriumhydroxyd 20 Stunden unter Rückfluss.

Nach Filtrieren zum Abtrennen der anorganischen Produkte wird das Reaktionsgemisch zur Trockne eingedampft und der Rückstand aus Aceton kristallisiert. Dabei erhält man die Verbindung der Formel:

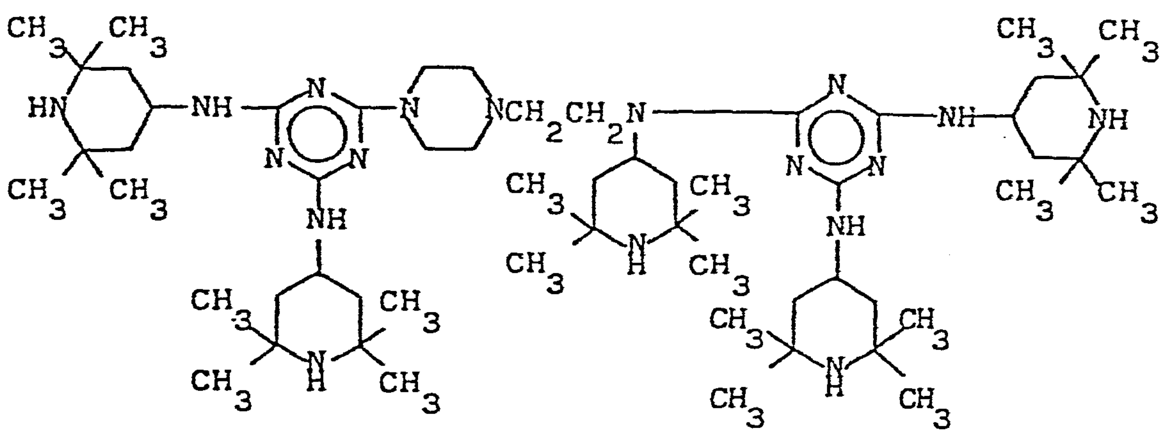

vom Schmelzpunkt 159-161°C.

Analyse für $C_{57}H_{106}N_{18}$

berechnet: C 65,60% H 10,24% N 24,16%
gefunden: C 65,44% H 10,37% N 23,64%

BEISPIEL 3

a) Herstellung von 2-Chlor-4,6-bis-[N-(2,2,6,6-tetramethyl-piperidin-4-yl)-äthylamino]-1,3,5-triazin.

Man gibt 36,8 g (0,2 Mol) 4-Aethylamino-2,2,6,6-tetramethyl-piperidin im Verlauf von 30 Minuten unter Kühlung, damit die Temperatur 40°C nicht übersteigt, zu einer Lösung von 18,5 g (0,1 Mol) Cyanurchlorid in 150 ml Xylol.

Dann setzt man 8 g in 20 ml Wasser gelöstes Natriumhydroxyd dazu und erhitzt 4 Stunden auf 60°C.

b) Umsetzung mit 1-[2-(2,2,6,6-Tetramethyl-piperidin-4-yl-amino)-äthyl]-piperazin.

Nach Abtrennung der wässrigen Phase von dem wie unter a) beschrieben erhaltenen Reaktionsgemisch gibt man 13,4 g (0,05 Mol) 1-[2-(2,2,6,6-Tetramethyl-piperidin-4-yl-amino)-äthyl]-piperazin und 6 g Natriumhydroxyd dazu und erhitzt 20 Stunden unter Rückfluss.

Nach Filtrieren zur Entfernung der anorganischen Produkte wird das Reaktionsgemisch zur Trockne eingedampft und der Rückstand aus Methanol kristallisiert.

Dabei erhält man die Verbindung der Formel

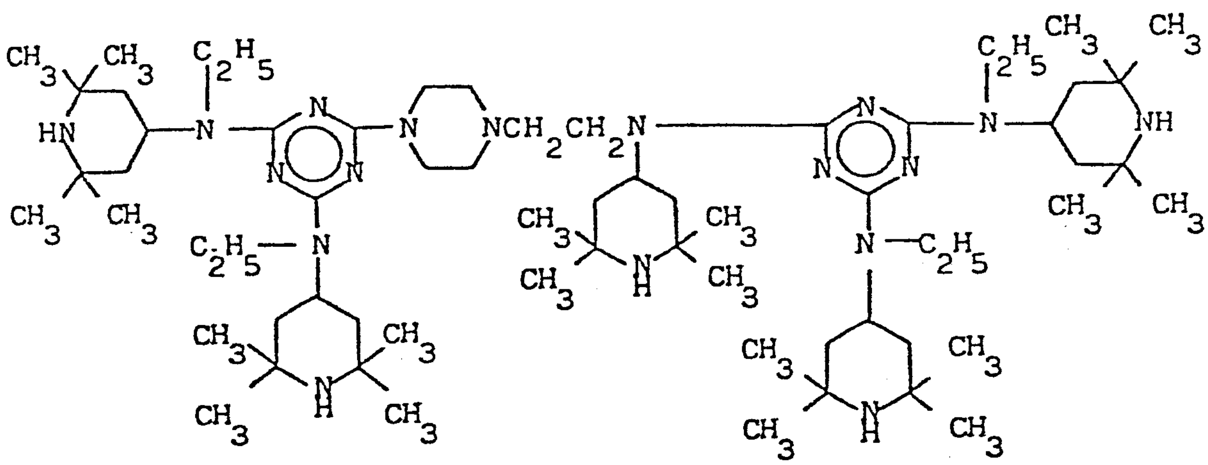

vom Schmelzpunkt 160°-161°C.

Analyse für $C_{65}H_{122}N_{18}$

berechnet: C 67,55% H 10,64% N 21,81%

gefunden: C 67,09% H 10,56% N 22,03%

BEISPIELE 4 und 5

Man verfährt wie in Beispiel 3 beschrieben, jedoch mit den entsprechenden Alkylaminoderivaten als Ausgangsprodukte, zur Herstellung der Verbindung der Formel

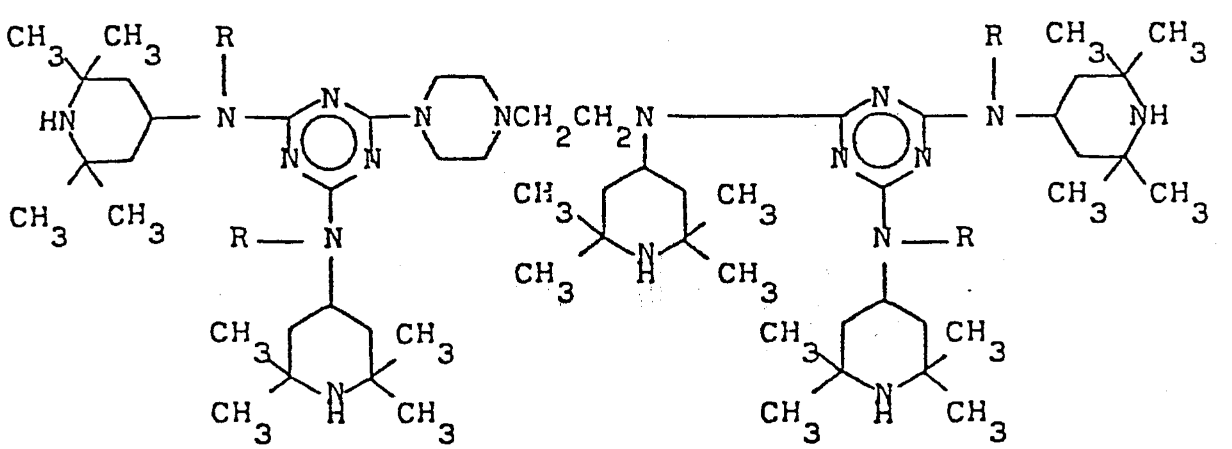

Die Definitionen für R und die Schmelzpunkte der erhaltenen Produkte sind in der nachfolgenden Tabelle angegeben:

| Beispiel Nr. | R | Schmelzpunkt °C |
|---|---|---|
| 4 | $n-C_4H_9-$ | 132-3 |
| 5 | $n-C_8H_{17}-$ | 125-6 |

BEISPIEL 6

Man gibt 13,4 g (0,05 Mol) 1-[2-(2,2,6,6-Tetramethylpiperidin-4-ylamino)-äthyl]-piperazin im Verlauf von 10 Minuten zu einer Lösung von 42,6 g (0,1 Mol) 2-Chlor-4,6-bis-(2,2,6,6-tetramethyl-piperidin-4-yloxy)-1,3,5-triazin in 250 ml Chloroform, rührt 2 Stunden bei Raumtemperatur und erhitzt dann 3 Stunden unter Rückfluss. Nach Abkühlung auf Raumtemperatur gibt man 4 g in 20 ml Wasser gelöstes Natriumhydroxyd dazu, rührt 1 Stunde bei derselben Temperatur, trennt die wässrige Phase ab, trocknet die organische Phase über $Na_2SO_4$ und dampft zur Trockne ein.

Der erhaltene Rückstand wird aus Hexan kristallisiert. Dabei erhält man die Verbindung der Formel

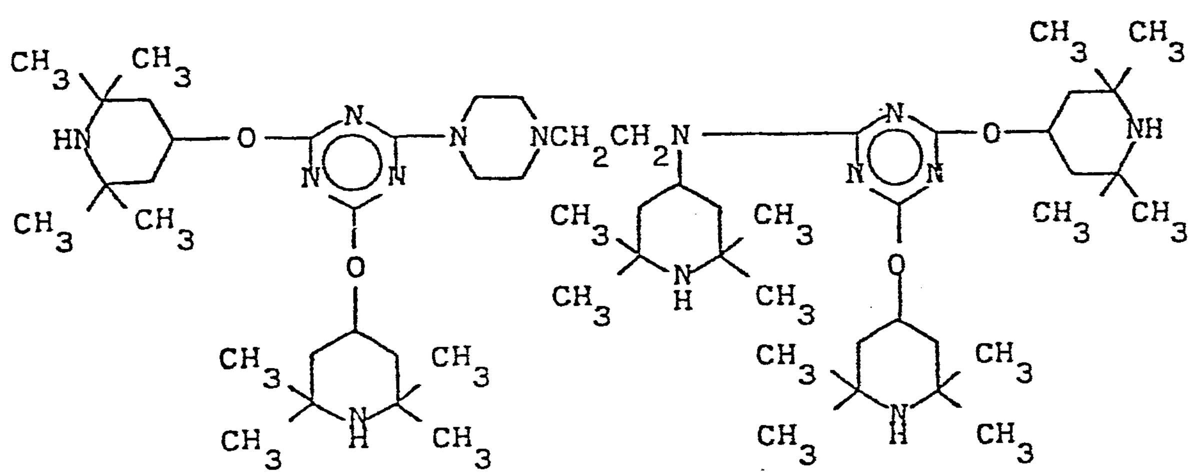

vom Schmelzpunkt 100-101°C.

Analyse für $C_{57}H_{102}N_{14}O_4$

berechnet: C 65,36% H 9,81% N 18,72% O 6,11%

gefunden: C 64,90% H 9,78% N 18,71%

BEISPIEL 7

Man erhitzt 22,2 g (0,1 Mol) 2-Butoxy-4,6-dichlor-1,3,5-triazin, 29,5 g (0,11 Mol) 1-[2-(2,2,6,6-Tetramethyl-piperidin-4-ylamino)-äthyl]-piperazin, 12 g Natriumhydroxyd und 150 ml Xylol 18 Stunden unter Rückfluss.

Nach Abtrennung der anorganischen Produkte und Abdampfung des Lösungsmittels erhält man ein harzartiges Produkt hellgelber Farbe, das ein zahlenmittleres Molekulargewicht von $\bar{M}n$ = 2 940 und einen Schmelzpunkt von 149-153°C besitzt.

BEISPIELE 8 - 11

Man verfährt wie in Beispiel 7 zur Herstellung der nachfolgenden Verbindungen durch Umsetzung eines Dichlortriazins mit 1-[2-(2,2,6,6-Tetramethyl-piperidin-4-ylamino)-äthyl]-piperazin (TMPP).

| Beispiel Nr. | Dichlortriazin (Mol) | TMPP (Mol) | $\bar{M}n$ | Schmelzpunkt (°C) |
|---|---|---|---|---|
| 8 | 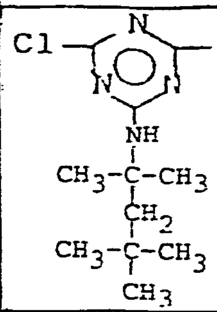 (0,1) | 0,11 | 2 600 | 154-8 |
| 9 | 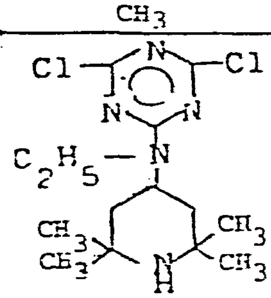 (0,1) | 0,11 | 3 120 | 207-212 |
| 10 | 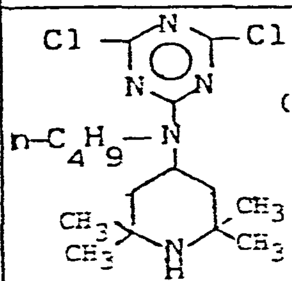 (0,1) | 0,11 | 3 050 | 175-80 |
| 11 | 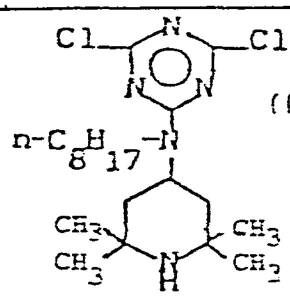 (0,1) | 0,11 | 3 430 | 150-54 |

Wie anfangs erwähnt, sind die Verbindungen der Formel (I) sehr wirksam bei der Verbesserung der Licht-, Wärme- und Oxydationsstabilität von synthetischen Polymeren, wie beispielsweise Polyäthylen hoher und niedriger Dichte, Polypropylen, Aethylen/Propylencopolymeren, Aethylen/Vinylacetatcopolymeren, Polybutadien, Polyisopren, Polystyrol, Butadien/Styrolcopolymeren, Vinylchlorid/Vinylidenchloridpolymeren und -copolymeren, Polyoxymethylen, Polyurethanen,

gesättigten und ungesättigten Polyestern, Polyamiden, Polycarbonaten, Polyacrylaten, Alkydharzen und Epoxidharzen.

Die Verbindungen der Formel (I) können im Gemisch mit synthetischen Polymeren in verschiedenen Anteilen eingesetzt werden, je nach der Art des Polymeren, dem Zweck und der Gegenwart weiterer Zusatzstoffe. Im allgemeinen setzt man zweckmässig 0,01 bis 5 Gew.-% der Verbindungen der Formel (I), bezogen auf das Gewicht der Polymeren, vorzugsweise 0,05 bis 1%, ein.

Die Verbindungen der Formel (I) können nach verschiedenen Verfahren in die polymeren Materialien eingearbeitet werden, wie durch trockenes Einmischen in Pulverform oder nasses Einmischen in Form von Lösungen oder Suspensionen oder auch in Form eines konzentrierten Stammgemischs; das synthetische Polymer lässt sich dabei in Form von Pulver, Granulat, einer Lösung, einer Suspension oder in Form eines Latex einsetzen.

Die mit den Produkten der Formel (I) stabilisierten Polymeren lassen sich zur Herstellung von Formkörpern, Folien, Bändern, Fasern, Endlosfäden, Lacken und dergleichen verwenden.

Gewünschtenfalls kann man den Gemischen der Verbindungen der Formel (I) mit den synthetischen Polymeren weitere Zusatzstoffe beigeben, wie beispielsweise:

1. Antioxidantien

1.1. Alkylierte Monophenole

2,6-Di-tert.butyl-4-methylphenol
2-Tert.butyl-4,6-dimethylphenol
2,6-Di-tert.butyl-4-ethylphenol
2,6-Di-tert.butyl-4-n-butylphenol
2,6-Di-tert.butyl-4-i-butylphenol
2,6-Di-cyclopentyl-4-methylphenol
2-(α-Methylcyclohexyl)-4,6-dimethylphenol
2,6-Di-octadecyl-4-methylphenol
2,4,6-Tri-cyclohexylphenol
2,6-Di-tert.butyl-4-methoxymethylphenol

1.2. Alkylierte Hydrochinone

2,6-Di-tert.butyl-4-methoxyphenol

2,5-Di-tert.butyl-hydrochinon

2,5-Di-tert.amyl-hydrochinon

2,6-Diphenyl-4-octadecyloxyphenol

1.3. Hydroxylierte Thiodiphenylether

2,2'-Thio-bis-(6-tert.butyl-4-methylphenol)

2,2'-Thio-bis-(4-octylphenol)

4,4'-Thio-bis-(6-tert.butyl-3-methylphenol)

4,4'-Thio-bis-(6-tert.butyl-2-methylphenol)

1.4. Alkyliden-Bisphenole

2,2'-Methylen-bis-(6-tert.butyl-4-methylphenol)

2,2'-Methylen-bis-(6-tert.butyl-4-ethylphenol)

2,2'-Methylen-bis-[4-methyl-6-(α-methylcyclohexyl)-phenol]

2,2'-Methylen-bis-(4-methyl-6-cyclohexylphenol)

2,2'-Methylen-bis-(6-nonyl-4-methylphenol)

2,2'-Methylen-bis-(4,6-di-tert.butylphenol)

2,2'-Ethyliden-bis-(4,6-di-tert.butylphenol)

2,2'-Ethyliden-bis-(6-tert.butyl-4-isobutylphenol)

2,2'-Methylen-bis-[6-(α-methylbenzyl)-4-nonylphenol]

2,2'-Methlyen-bis-[6-(α,α-dimethylbenzyl)-4-nonylphenol]

4,4'-Methylen-bis-(2,6-di-tert.butylphenol)

4,4'-Methylen-bis-(6-tert.butyl-2-methylphenol)

1,1-Bis-(5-tert.butyl-4-hydroxy-2-methylphenyl)-butan

2,6-Di-(3-tert.butyl-5-methyl-2-hydroxybenzyl)-4-methylphenol

1,1,3-Tris-(5-tert.butyl-4-hydroxy-2-methylphenyl)-butan

1,1-Bis-(5-tert.butyl-4-hydroxy-2-methylphenyl)-3-n-dodecylmercapto-butan

Ethylenglycol-bis-[3,3-bis-(3'-tert.butyl-4'-hydroxyphenyl)-butyrat]

Di-(3-tert.butyl-4-hydroxy-5-methylphenyl)-dicyclopentadien

Di-[2-(3'-tert.butyl-2'-hydroxy-5'-methyl-benzyl)-6-tert.butyl-4-methyl-phenyl]-terephthalat.

1.5 Benzylverbindungen

1,3,5-Tri-(3,5-di-tert.butyl-4-hydroxybenzyl)-2,4,6-trimethylbenzol

Di-(3,5-di-tert.butyl-4-hydroxybenzyl)-sulfid

3,5-di-tert.butyl-4-hydroxybenzyl-mercaptoessigsäure-isooctylester
Bis-(4-tert.butyl-3-hydroxy-2,6-dimethylbenzyl)dithiol-terephthalat
1,3,5-Tris-(3,5-di-tert.butyl-4-hydroxybenzyl)-isocyanurat
1,3,5-Tris-(4-tert.butyl-3-hydroxy-2,6-dimethylbenzyl)-isocyanurat
3,5-Di-tert.butyl-4-hydroxybenzyl-phosphonsäure-dioctadecylester
3,5-Di-tert.butyl-4-hydroxybenzyl-phosphonsäure-monoethylester, Calcium-salz.

1.6. Acylaminophenole
4-Hydroxy-laurinsäureanilid
4-Hydroxy-stearinsäureanilid
2,4-Bis-octylmercapto-6-(3,5-di-tert.butyl-4-hydroxyanilino)-s-triazin
N-(3,5-di-tert.butyl-4-hydroxyphenyl)-carbaminsäureoctylester.

1.7. Ester der ß-(3,5-Di-tert.butyl-4-hydroxyphenyl)-propionsäure mit ein- oder mehrwertigen Alkoholen, wie z.B. mit

| | |
|---|---|
| Methanol | Diethylenglycol |
| Octadecanol | Triethylenglycol |
| 1,6-Hexandiol | Pentaerythrit |
| Neopentylglycol | Tris-hydroxyethyl-isocyanurat |
| Thiodiethylenglycol | Di-hydroxyethyl-oxalsäurediamid |

1.8. Ester der ß-(5-tert.butyl-4-hydroxy-3-methylphenyl)-propionsäure mit ein- oder mehrwertigen Alkoholen, wie z.B. mit

| | |
|---|---|
| Methanol | Diethylenglycol |
| Octadecanol | Triethylenglycol |
| 1,6-Hexandiol | Pentaerythrit |
| Neopentylglycol | Tris-hydroxyethyl-isocyanurat |
| Thiodiethylenglycol | Di-hydroxyethyl-oxalsäurediamid |

1.9. Amide der ß-(3,5-Di-tert.butyl-4-hydroxyphenyl)-propionsäure, wie z.B.
N,N'-Di-(3,5-di-tert.butyl-4-hydroxyphenylpropionyl)-hexamethylendiamin

N,N'-Di-(3,5-di-tert.butyl-4-hydroxyphenylpropionyl)-trimethylen-diamin
N,N'-Di-(3,5-di-tert.butyl-4-hydroxyphenylpropionyl)-hydrazin

2. UV-Absorber und Lichtschutzmittel

2.1. 2-(2'-Hydroxyphenyl)-benztriazole, wie z.B. das 5'-Methyl-, 3',5'-Di-tert.butyl-, 5'-Tert.butyl-, 5'-(1,1,3,3-Tetramethylbutyl)-, 5-Chlor-3',5'-di-tert.butyl-, 5-Chlor-3'-tert.butyl-5'-methyl-, 3'-sec.Butyl-5'-tert.butyl, 4'-Octoxy-, 3',5'-Di-tert.amyl-, 3',5'-Bis-(α,α-dimethylbenzyl)-Derivat.

2.2. 2-Hydroxybenzophenone, wie z.B. das 4-Hydroxy-, 4-Methoxy-, 4-Octoxy-, 4-Decyloxy-, 4-Dodecyloxy-, 4-Benzyloxy-, 4,2',4'-Trihydroxy-, 2'-Hydroxy-4,4'-dimethoxy-Derivat.

2.3. Ester von gegebenenfalls substituierten Benzoesäuren, wie z.B. 4-Tert.butyl-phenylsalicylat, Phenylsalicylat, Octylphenylsalicylat, Dibenzoylresorcin, Bis-(4-tert.butylbenzoyl)-resorcin, Benzoylresorcin, 3,5-Di-tert.butyl-4-hydroxybenzoesäure-2,4-di-tert.butylphenylester, 3,5-Di-tert.butyl-4-hydroxybenzoesäurehexadecylester.

2.4. Acrylate, wie z.B. α-Cyan-ß,ß-diphenylacrylsäure-ethylester bzw. -isooctylester, α-Carbomethoxy-zimtsäuremethylester, α-Cyano-ß-methyl-p-methoxy-zimtsäuremethylester bzw. -butylester, α-Carbomethoxy-p-methoxy-zimtsäure-methylester, N-(ß-Carbomethoxy-ß-cyanovinyl)-2-methyl-indolin,

2.5. Nickelverbindungen, wie z.B. Nickelkomplexe des 2,2'-Thio-bis-[4-(1,1,3,3-tetramethylbutyl)-phenols], wie der 1:1- oder der 1:2-Komplex, gegebenenfalls mit zusätzlichen Liganden wie n-Butylamin, Triethanolamin oder N-Cyclohexyl-diethanolamin, Nickeldibutyldithiocarbamat, Nickelsalze von 4-Hydroxy-3,5-di-tert.butylbenzylphosphonsäure-monoalkylestern wie vom Methyl- oder Ethylester, Nickelkomplexe von Ketoximen wie von 2-Hydroxy-4-methyl-phenyl-undecylketonoxim, Nickelkomplexe des 1-Phenyl-4-lauroyl-5-hydroxy-pyrazols, gegebenenfalls mit zusätzlichen Liganden.

2.6 Sterisch gehinderte Amine, wie z.B. Bis-(2,2,6,6-tetramethylpiperidyl)-sebacat
Bis-(1,2,2,6,6-pentamethylpiperidyl)-sebacat
n-Butyl-3,5-di-tert.butyl-4-hydroxybenzyl-malonsäure-bis(1,2,2,6,6-pentamethylpiperidyl)-ester, Kondensationsprodukt aus 1-Hydroxyethyl-2,2,6,6-Tetramethyl-4-hydroxypiperidin und Bernsteinsäure, Kondensationsprodukt aus N,N'-(2,2,6,6-Tetramethylpiperidyl)-hexamethylendiamin und 4-tert.octylamino-2,6-dichlor-1,3,5-s-triazin,
Tris-(2,2,6,6-tetramethylpiperidyl)-nitrilotriacetat,
Tetrakis-(2,2,6,6-tetramethyl-4-piperidyl)-1,2,3,4-butantetracarbonsäure,
1,1'-(1,2-ethandiyl)-bis-(3,3,5,5-tetramethyl-piperazinon).

2.7. Oxalsäurediamide, wie z.B. 4,4'-Di-octyloxy-oxanilid, 2,2'-Di-octyloxy-5,5'-di-tert.butyl-oxanilid, 2,2'-Di-dodecyloxy-5,5'-di-tert.butyl-oxanilid, 2-Ethoxy-2'-ethyl-oxanilid, N,N'-Bis-(3-dimethylaminopropyl)-oxalamid, 2-Ethoxy-5-tert.butyl-2'-ethyl-oxanilid und dessen Gemisch mit 2-Ethoxy-2'-ethyl-5,4'-di-tert.butyl-oxanilid, Gemische von ortho-und para-Methoxy- sowie von o- und p-Ethoxy-di-substituierte Oxaniliden.

3. Metalldesaktivatoren, wie z.B. N,N'-Diphenyloxalsäurediamid, N-Salicylal-N'-salicyloylhydrazin, N,N'-Bis-salicyloylhydrazin, N,N'-Bis-(3,5-di-tert.butyl-4-hydroxyphenylpropionyl)-hydrazin, 3-Salicyloylamino-1,2,4-triazol, Bis-benzyliden-oxalsäuredihydrazid.

4. Phosphite und Phosphonite, wie z.B. Triphenylphosphit, Diphenylalkylphosphite, Phenyldialkylphosphite, Tri-(nonylphenyl)-phosphit, Trilaurylphosphit, Trioctadecylphosphit, Distearyl-pentaerythritdiphosphit, Tris-(2,4-di-tert.butylphenyl)-phosphit, Diisodecylpentaerythrit-diphosphit, Di-(2,4-di-tert.butylphenyl)-pentaerythritdiphosphit, Tristearyl-sorbit-triphosphit, Tetrakis-(2,4-di-tert.butylphenyl)-4,4'-biphenylen-diphosphonit.

5. Peroxidzerstörende Verbindungen, wie z.B. Ester der ß-Thio-dipropionsäure, beispielsweise der Lauryl-, Stearyl-, Myristyl- oder

Tridecylester, Mercaptobenzimidazol, das Zinksalz des 2-Mercaptobenzimidazols, Zink-dibutyl-dithiocarbamat, Dioctadecyldisulfid, Pentaerythrit-tetrakis-(ß-dodecylmercapto)-propionat.

6. Polyamidstabilisatoren, wie z.B. Kupfersalze in Kombination mit Jodiden und/oder Phosphorverbindungen und Salze des zweiwertigen Mangans.

7. Basische Co-Stabilisatoren, wie z.B. Melamin, Polyvinylpyrrolidon, Dicyandiamid, Triallylcyanurat, Harnstoff-Derivate, Hydrazin-Derivate, Amine, Polyamide, Polyurethane, Alkali- und Erdalkalisalze höherer Fettsäuren, beispielsweise Ca-Stearat, Zn-Stearat, Mg-Stearat, Na-Ricinoleat, K-Palmitat, Antimonbrenzcatechinat oder Zinnbrenzcatechinat.

8. Nukleierungsmittel, wie z.B. 4-tert.Butylbenzoesäure, Adipinsäure, Diphenylessigsäure.

9. Füllstoffe und Verstärkungsmittel, wie z.B. Calciumcarbonat, Silikate, Glasfasern, Asbest, Talk, Kaolin, Glimmer, Bariumsulfat, Metalloxide und -hydroxide, Russ, Graphit.

10. Sonstige Zusätze, wie z.B. Weichmacher, Gleitmittel, Emulgatoren, Pigmente, Optische Aufheller, Flammschutzmittel, Antistatika, Treibmittel.

Die Wirksamkeit der erfindungsgemäss hergestellten Produkte als Stabilisatoren wird in den nachfolgenden Beispielen erläutert, wo einige der in den Herstellungsbeispielen erhaltenen Produkte zur Stabilisierung von Polypropylenbändern und -fasern und Folien aus Polyäthylen niedriger Dichte eingesetzt werden.

BEISPIEL 12

Bei den in Tabelle 1 angeführten Prüfungen werden je 2 g eines der erfindungsgemässen Stabilisatoren, 1 g Pentaerythrit-tetrakis-3-(3,5-di-t-butyl-4-hydroxyphenyl)-propionat (Antioxydans) und 1 g Calciumstearat in einem langsam laufenden Mischer mit 1 000 g Polypropylenpulver vom

Schmelzindex 2,4 ([R]Propathene HF 18, einem Produkt der Imperial Chemical Industries) vermischt.

Das Gemisch wird bei einer Temperatur von 200°-220°C extrudiert und zu Granulat verarbeitet, aus dem man dann Bänder einer Dicke von 50 μm und einer Breite von 2,5 mm unter den folgenden Arbeitsbedingungen herstellt:

| | |
|---|---|
| Extrudertemperatur | : 220°-240°C |
| Kopftemperatur | : 240°C |
| Streckverhältnis | : 1:6. |

Die so erhaltenen Bänder werden auf weissem Karton in einem Weather-Ometer 65 WR (ASTM G27-70) bei einer Temperatur der schwarzen Tafel von 63°C ausgesetzt. Von Zeit zu Zeit werden Proben entnommen, an denen man die verbleibende Reissfestigkeit mittels eines Dynamometers mit konstanter Geschwindigkeit misst; danach berechnet man die zum Halbieren der ursprünglichen Reissfestigkeit erforderliche Aussetzungsperiode ($T_{50}$).

Zum Vergleich werden unter denselben Bedingungen wie oben angegeben, jedoch ohne den Zusatz der erfindungsgemässen Verbindungen hergestellte Polypropylenbänder ausgesetzt.

Die erhaltenen Ergebnisse sind in Tabelle 1 angeführt:

TABELLE 1

| Stabilisator | $T_{50}$ (Stunden) |
|---|---|
| Keiner | 220 |
| Verbindung aus Beispiel 2 | 1 330 |
| Verbindung aus Beispiel 3 | 1 940 |
| Verbindung aus Beispiel 4 | 1 650 |
| Verbindung aus Beispiel 5 | 1 570 |
| Verbindung aus Beispiel 6 | 1 580 |
| Verbindung aus Beispiel 7 | 1 030 |
| Verbindung aus Beispiel 8 | 1 000 |
| Verbindung aus Beispiel 9 | 1 200 |
| Verbindung aus Beispiel 10 | 1 800 |
| Verbindung aus Beispiel 11 | 1 260 |

BEISPIEL 13

Bei den in Tabelle 2 angeführten Prüfungen werden

je 2,5 g eines der erfindungsgemässen Stabilisatoren, 0,5 g n-Octadecyl-3-(3,5-di-t-butyl-4-hydroxyphenyl)-propionat (Antioxydans) und 1 g Calciumstearat in einem langsam laufenden Mischer mit 1 000 g Polypropylenpulver vom Schmelzindex 13 (®Propathene HF 85, einem Produkt der Imperial Chemical Industries) vermischt. Das Gemisch wird dann bei einer Temperatur von 200°-220°C zu Granulat extrudiert, das man dann unter den folgenden Arbeitsbedingungen zu Fasern verarbeitet:

| | |
|---|---|
| Extrudertemperatur | : 220°-250°C |
| Spinndüsentemperatur | : 240°C |
| Streckverhältnis | : 1:3,5 |
| Titer | : 20 denier pro Faser |

Die erhaltenen Fasern werden auf weissem Karton in einem Weather-Ometer 65 WR (ASTM G27-70) bei einer Temperatur der schwarzen Tafel von 63°C ausgesetzt. Dann wird der $T_{50}$-Wert wie in Beispiel 12 beschrieben berechnet.

Zum Vergleich sind die mit unter denselben Bedingungen wie oben, aber ohne den Zusatz der erfindungsgemässen Verbindungen hergestellten Fasern erhaltenen Daten ebenfalls angegeben.

Die erhaltenen Ergebnisse sind in Tabelle 2 angeführt:

TABELLE 2

| Stabilisator | $T_{50}$ (Stunden) |
|---|---|
| Keiner | 180 |
| Verbindung aus Beispiel 4 | 1 410 |
| Verbindung aus Beispiel 7 | 1 000 |
| Verbindung aus Beispiel 8 | 1 080 |
| Verbindung aus Beispiel 9 | 1 200 |
| Verbindung aus Beispiel 10 | 1 640 |
| Verbindung aus Beispiel 11 | 1 100 |

BEISPIEL 14

Bei den in Tabelle 3 angeführten Prüfungen werden je 2 g eines der erfindungsgemässen Stabilisatoren und 0,3 g Octadecyl-3-(3,5-di-t-butyl-4-hydroxyphenyl)-propionat (Antioxydans) mit 1 000 g Polyäthylenpulver niedriger Dichte vom Schmelzindex 0,6 (®Fertene EF 3-2000, einem Produkt der Soc. Montedison) innig vermischt.

Das so erhaltene Gemisch wird dann bei einer Temperatur von 190°C extrudiert und zu Granulat verarbeitet, aus dem durch Verpressen bei 200°C 0,2 mm dicke Folien hergestellt werden, die man dann auf weissem Karton in einem Weather-Ometer 65 WR (ASTM G27-70) mit einer Temperatur der schwarzen Tafel von 63°C aussetzt.

An den ausgesetzten Proben wird die zum Anstieg des Carbonylgruppengehalts bei 5,85 Mikrometer um 0,2% erforderliche Zeit ($T_{0,2}$) bestimmt.

Zum Vergleich wird eine Polymerfolie ohne Zusatz erfindungsgemässer Stabilisatoren hergestellt und unter denselben Bedingungen wie oben beschrieben belichtet.

Die erhaltenen Ergebnisse sind in Tabelle 3 angeführt:

TABELLE 3

| Stabilisator | $T_{0,2}$ (Stunden) |
|---|---|
| Keiner | 900 |
| Verbindung aus Beispiel 4 | 5 700 |
| Verbindung aus Beispiel 7 | 3 940 |
| Verbindung aus Beispiel 8 | 5 300 |
| Verbindung aus Beispiel 9 | 5 800 |
| Verbindung aus Beispiel 10 | 4 500 |
| Verbindung aus Beispiel 11 | 4 900 |

Patentansprüche:

1. Verbindungen der allgemeinen Formel (I)

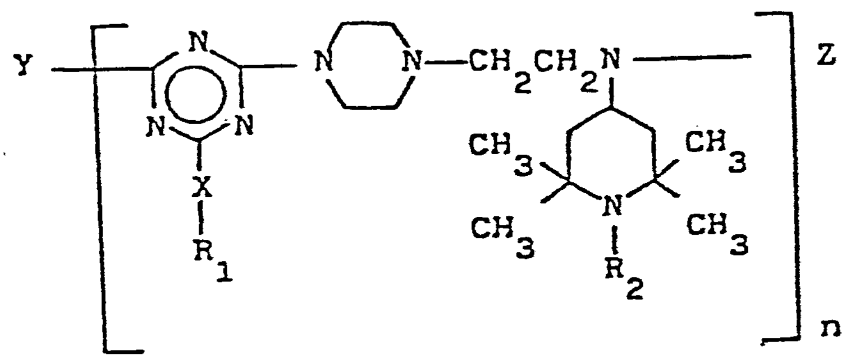

(I)

worin $R_1$ für Wasserstoff, $C_1$-$C_{18}$-Alkyl, $C_3$-$C_{18}$-Alkenyl, $C_5$-$C_{18}$-Cycloalkyl, unsubstituiertes oder durch 1 bis 3 $C_1$-$C_8$-Alkylreste substituiertes Phenyl, unsubstituiertes oder durch 1 bis 3 $C_1$-$C_8$-Alkylreste substituiertes Hydroxyphenyl, unsubstituiertes oder durch 1 bis 3 $C_1$-$C_4$-Alkylreste substituiertes Benzyl oder einen Rest $-R_3-Z'-$, in dem $R_3$ $C_2$-$C_6$-Alkylen und Z' $-OR_4$ oder $-\underset{\substack{|\\R_5}}{N}-R_6$ bedeuten, wobei $R_4$ Wasserstoff oder $C_1$-$C_{18}$-Alkyl sowie $R_5$ und $R_6$, die gleich oder verschieden sein können, $C_1$-$C_8$-Alkyl oder $C_3$-$C_8$-Alkenyl darstellen oder $-\underset{\substack{|\\R_5}}{N}-R_6$ als ganzes Morpholin-4-yl, 4-Methyl-piperazin-1-yl oder 2,2,6,6-Tetramethyl-piperidin-1-yl sein kann, oder für einen Rest der Formel (II)

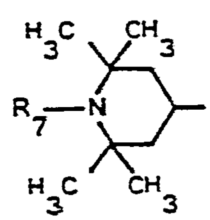

(II)

steht, worin $R_7$ Wasserstoff, O·, Cyanmethyl, $C_1$-$C_{12}$-Alkyl, $C_3$-$C_{12}$-Alkenyl oder -alkinyl, unsubstituiertes oder durch 1 bis 3 $C_1$-$C_4$-Alkylreste substituiertes Benzyl, unsubstituiertes oder durch 1 bis 3 $C_1$-$C_4$-Alkylreste substituiertes Hydroxybenzyl $C_1$-$C_{12}$-Acyl, 2,3-Epoxypropyl oder $-CH_2-\underset{\substack{|\\R_8}}{CH}-OR_9$ bedeutet, worin

$R_8$ Wasserstoff, Methyl, Aethyl oder Phenyl und $R_9$ Wasserstoff, $C_1$-$C_{12}$-Alkyl, $C_3$-$C_{12}$-Alkenyl, $C_7$-$C_{12}$-Aralkyl oder $C_1$-$C_{12}$-Acyl darstellen, $R_2$ die für $R_7$ angegebene Bedeutung hat, X für -O- oder $-\underset{R_{10}}{\underset{|}{N}}-$ steht, wobei $R_{10}$ die für $R_1$ angegebene Bedeutung hat, oder $R_1X$- als ganzes auch für Chlor, Pyrrolidin-1-yl, Piperidin-1-yl, Hexahydroazepin-1-yl, Morpholin-4-yl, 4-Methyl-piperazin-1-yl oder einen Rest der Formel (IIIa) oder (IIIb)

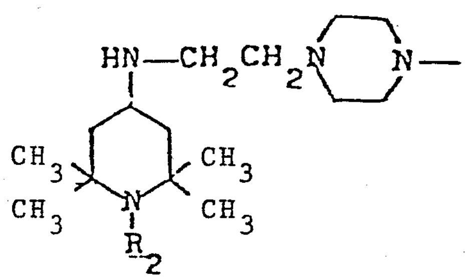

(IIIa)

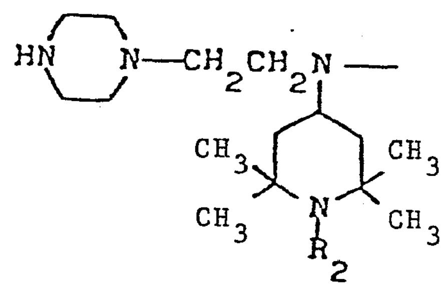

(IIIb)

stehen kann, worin $R_2$ die oben angegebene Bedeutung hat, Y für Chlor, einen Rest $R_1X$-, worin $R_1$ und X die oben angegebenen Bedeutungen haben, oder einen Rest der Formel (IIIa) oder (IIIb) und Z für Wasserstoff oder einen Rest der Formel (IV)

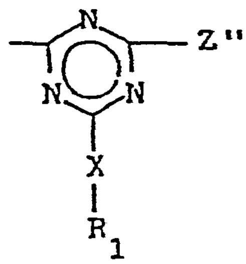

(IV)

stehen, worin Z" Chlor oder einen Rest $R_1X$- darstellt, worin $R_1$ und X die oben angegebenen Bedeutungen haben, und n eine ganze Zahl von 1 bis 100 ist.

2. Verbindungen der Formel (I) nach Anspruch 1, dadurch gekennzeichnet, dass $R_1$ und $R_{10}$, die gleich oder verschieden sein können, für Wasserstoff, $C_1$-$C_{12}$-Alkyl, $C_6$-$C_{12}$-Cycloalkyl, 2,2,6,6-Tetramethyl-piperidin-4-yl oder 1,2,2,6,6-Pentamethyl-piperidin-4-yl, $R_2$ und $R_7$, die gleich oder verschieden sein können, für Wasserstoff, $C_1$-$C_6$-Alkyl, $C_3$-$C_6$-Alkenyl oder -alkinyl, Benzyl, $C_1$-$C_6$-Alkanoyl, 2,3-Epoxypropyl oder 2-Hydroxyäthyl oder $R_1X$- als ganzes für Chlor, Piperidin-1-yl, Hexahydroazepin-1-yl, Morpholin-4-yl oder einen Rest der Formel (IIIa) oder (IIIb) stehen und n eine ganze Zahl von 1 bis 50 ist.

3. Verbindungen der Formel (I) nach Anspruch 1, dadurch gekennzeichnet, dass $R_1$ für Wasserstoff, $C_1$-$C_8$-Alkyl, 2,2,6,6-Tetramethyl-piperidin-4-yl oder 1,2,2,6,6-Pentamethyl-piperidin-4-yl, $R_2$ und $R_7$ für Wasserstoff oder Methyl und $R_{10}$ für Wasserstoff oder $C_1$-$C_8$-Alkyl stehen, n eine ganze Zahl von 1 bis 20 ist und Y für einen Rest $R_1X$- oder einen Rest (IIIa) oder (IIIb) und Z für Wasserstoff oder einen Rest

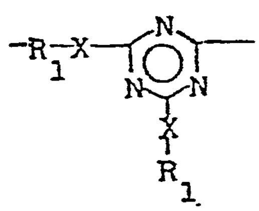

stehen.

4. Verfahren zur Herstellung von Verbindungen der Formel (I)

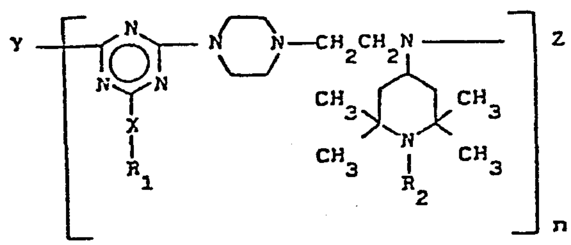

worin $R_1$ für Wasserstoff, $C_1$-$C_{18}$-Alkyl, $C_3$-$C_{18}$-Alkenyl, $C_5$-$C_{18}$-Cycloalkyl, unsubstituiertes oder durch 1 bis 3 $C_1$-$C_8$-Alkylreste substituiertes Phenyl, unsubstituiertes oder durch 1 bis 3 $C_1$-$C_8$-Alkylreste substituiertes Hydroxyphenyl, unsubstituiertes oder durch 1 bis 3 $C_1$-$C_4$-Alkylreste substituiertes Benzyl oder einen Rest $-R_3-Z'-$, in dem $R_3$ $C_2$-$C_6$-Alkylen und Z' $-OR_4$ oder $-\underset{R_5}{\underset{|}{N}}-R_6$ bedeuten, wobei $R_4$ Wasserstoff oder $C_1$-$C_{18}$-Alkyl sowie $R_5$ und $R_6$, die gleich oder verschieden sein können, $C_1$-$C_8$-Alkyl oder $C_3$-$C_8$-Alkenyl darstellen oder $-\underset{R_5}{\underset{|}{N}}-R_6$ als ganzes Morpholin-4-yl, 4-Methyl-piperazin-1-yl oder 2,2,6,6-Tetramethyl-piperidin-1-yl sein kann oder für einen Rest der Formel (II)

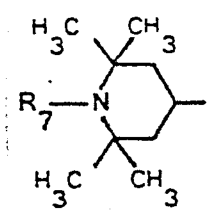

(II)

steht, worin $R_7$ Wasserstoff, O•, Cyanmethyl, $C_1$-$C_{12}$-Alkyl, $C_3$-$C_{12}$-Alkenyl oder -alkinyl, unsubstituiertes oder durch 1 bis 3 $C_1$-$C_4$-Alkylreste substituiertes Benzyl, unsubstituiertes oder durch 1 bis 3 $C_1$-$C_4$-Alkylreste substituiertes Hydroxybenzyl, $C_1$-$C_{12}$-Acyl, 2,3-Epoxypropyl oder $-CH_2-\underset{R_8}{\underset{|}{CH}}-OR_9$ bedeutet, worin $R_8$ Wasserstoff, Methyl, Aethyl oder Phenyl und $R_9$ Wasserstoff, $C_1$-$C_{12}$-Alkyl, $C_3$-$C_{12}$-Alkenyl, $C_7$-$C_{12}$-Aralkyl oder

$C_1$-$C_{12}$-Acyl darstellen, $R_2$ die für $R_7$ angegebene Bedeutung hat, X für -O- oder $\underset{\displaystyle R_{10}}{-\overset{}{N}-}$ steht, wobei $R_{10}$ die für $R_1$ angegebene Bedeutung hat, oder $R_1X$- als ganzes auch für Chlor, Pyrrolidin-1-yl, Piperidin-1-yl, Hexahydroazepin-1-yl, Morpholin-4-yl, 4-Methyl-piperazin-1-yl oder einen Rest der Formel (IIIa) oder (IIIb)

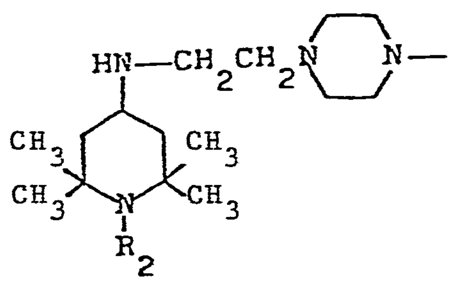

(IIIa)

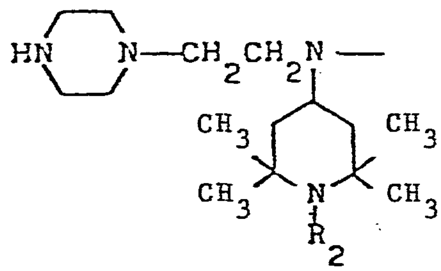

(IIIb)

stehen kann, worin $R_2$ die oben angegebene Bedeutung hat, Y für Chlor, einen Rest $R_1X$-, worin $R_1$ und X die oben angegebenen Bedeutungen haben, oder einen Rest der Formel (IIIa) oder (IIIb) und Z für Wasserstoff oder einen Rest der Formel (IV)

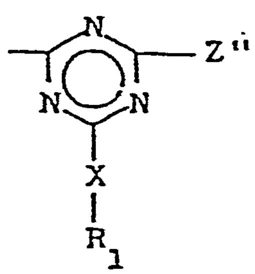

(IV)

stehen, worin Z" Chlor oder einen Rest $R_1X$- darstellt, worin

$R_1$ und X die oben angegebenen Bedeutungen haben, und n eine ganze Zahl von 1 bis 100 ist, dadurch gekennzeichnet, dass man eine Verbindung der Formel (V)

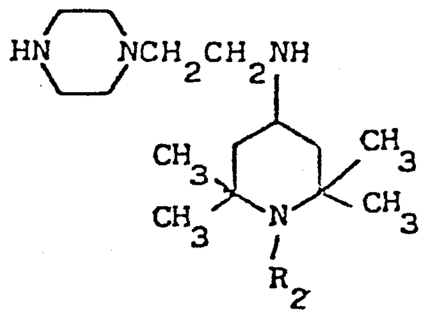

(V)

mit Cyanurchlorid oder dessen teilweise substituierten Derivaten der Formel (VI) oder (VII)

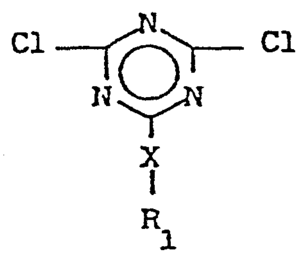

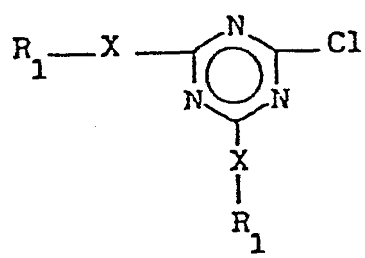

(VI) (VII)

worin $R_1X$- von Chlor verschieden ist, und mit einer Verbindung der Formel (X)

$$R_1\text{-X-H} \qquad (X)$$

in einer Stufe oder in zwei aufeinanderfolgenden Stufen bei einer Temperatur zwischen -20° und 200°C sowie in Gegenwart eines inerten Lösungsmittels oder einer organischen oder anorganischen Base umsetzt.

5. Licht-, wärme- und oxydationstabilisierte Polymerzusammensetzungen, dadurch gekennzeichnet, dass sie aus einem synthetischen Polymeren und einem oder mehreren Stabilisatoren der Formel (I) in einer Menge von 0,01 bis 5 Gew.-%, vorzugsweise von 0,05 bis 1 Gew.-%, bezogen auf das Gewicht des synthetischen Polymeren, bestehen.

6. Zusammensetzungen nach Anspruch 5, dadurch gekennzeichnet, dass sie zusätzlich zu dem neuen Stabilisator der Formel (I) weitere übliche Zusatzstoffe für synthetische

Polymere enthalten.

7. Zusammensetzungen nach Anspruch 5, dadurch gekennzeichnet, dass als synthetisches Polymer Polyäthylen oder Polypropylen vorliegt.

Europäisches Patentamt

**EUROPÄISCHER RECHERCHENBERICHT**

0117229 Nummer der Anmeldung

EP 84 81 0055

## EINSCHLÄGIGE DOKUMENTE

| Kategorie | Kennzeichnung des Dokuments mit Angabe, soweit erforderlich, der maßgeblichen Teile | Betrifft Anspruch | KLASSIFIKATION DER ANMELDUNG (Int. Cl. ³) |
|---|---|---|---|
| X | US-A-4 263 434 (CHIMOSA CHIMICA ORGANICA S.p.A.)<br>* Ansprüche 8,9 *<br>--- | 1,5,6 | C 07 D 401/14<br>C 08 K 5/34 |
| A | EP-A-0 053 775 (HOECHST AG)<br>* Beispiel 23 *<br>--- | 1,5 | |
| A | EP-A-0 042 554 (HOECHST AG)<br>* Ansprüche *<br>--- | 1,5 | |
| A | EP-A-0 070 358 (AMERICAN CYANAMID)<br>* Ansprüche *<br>----- | 1,5 | |
| | | | RECHERCHIERTE SACHGEBIETE (Int. Cl. ³) |
| | | | C 07 D 401/00 |

Der vorliegende Recherchenbericht wurde für alle Patentansprüche erstellt.

| Recherchenort | Abschlußdatum der Recherche | Prüfer |
|---|---|---|
| DEN HAAG | 02-05-1984 | VAN BIJLEN H. |

KATEGORIE DER GENANNTEN DOKUMENTEN

X : von besonderer Bedeutung allein betrachtet
Y : von besonderer Bedeutung in Verbindung mit einer anderen Veröffentlichung derselben Kategorie
A : technologischer Hintergrund
O : nichtschriftliche Offenbarung
P : Zwischenliteratur
T : der Erfindung zugrunde liegende Theorien oder Grundsätze

E : älteres Patentdokument, das jedoch erst am oder nach dem Anmeldedatum veröffentlicht worden ist
D : in der Anmeldung angeführtes Dokument
L : aus andern Gründen angeführtes Dokument

& : Mitglied der gleichen Patentfamilie, übereinstimmendes Dokument

EPA Form 1503 03 82